# EUROPEAN PATENT SPECIFICATION

(11) **EP 2 224 054 B1**
(45) Date of publication and mention of the grant of the patent: **14.03.2012**
(21) Application number: 08861220.5
(22) Date of filing: 17.12.2008
(51) Int. Cl.: D06M 10/00, A61L 2/16, A61L 2/238, D06M 11/42, D06M 11/49, D06M 11/83, D06M 23/08

(54) **METHOD FOR ANTIMICROBIAL TREATMENT OF FIBER, PROCESS FOR PRODUCTION OF ANTIMICROBIAL FIBER, AND ANTIMICROBIAL FIBER**
VERFAHREN ZUR ANTIMIKROBIELLEN AUSRÜSTUNG EINER FASER, VERFAHREN ZUR HERSTELLUNG EINER ANTIMIKROBIELL AUSGERÜSTETEN FASER SOWIE ANTIMIKROBIELL AUSGERÜSTETE FASER
PROCÉDÉ POUR TRAITEMENT ANTIMICROBIEN DE FIBRE, PROCESSUS DE PRODUCTION DE FIBRE ANTIMICROBIENNE, ET FIBRE ANTIMICROBIENNE

(30) Priority: 19.12.2007 JP 2007327645
(43) Date of publication of application: 01.09.2010
(73) Proprietor: Osaka University, Suita-shi Osaka 565-0871 (JP)
(72) Inventor: SEINO, Satoshi, Suita-shi Osaka 565-0871 (JP); IDE, Masato, Suita-shi Osaka 565-0871 (JP); UEDA, Hiroyuki, Suita-shi Osaka 565-0871 (JP); UJIIE, Kadumasa, Suita-shi Osaka 565-0871 (JP); KADOBAYASHI, Atsushi, Suita-shi Osaka 565-0871 (JP)
(74) Representative: Duckett, Anthony Joseph
(86) International application number: PCT/JP2008/072997
(87) International publication number: WO 2009/078442

(56) References cited:
- WO-A1-2006/126783
- WO-A2-2007/074484
- JP-A- 2004 084 153
- JP-A- 2005 126 348
- JP-A- 2005 296 079
- US-A1- 2007 166 399

## Description

### Technical Field

The present invention relates to an antibacterial treatment method for fiber, a method for producing antibacterial fiber and antibacterial fiber. More particularly, it relates to an antibacterial treatment method for fiber in which antibacterial metal particulates are firmly adhered to the fiber, a method for producing antibacterial fiber and an antibacterial fiber produced by the production method.

### Background Art

Harm caused by resistant bacteria such as MRSA derived from frequent use of antibiotics is recently becoming a problem, and new merits have been discovered in inorganic bactericides and antibacterial agents. This is principally because a metal such as silver or a metal oxide such as titanium oxide does not always exhibit a strong bactericidal function but is harmless to the human body without producing resistant bacteria and exhibits continuous antibacterial/antimold function against a wide range of bacteria and mold.

Antibacterial agents using antibacterial metals are already widely commercially available as products. Examples of an antibacterial agent using silver as an antibacterial metal are known as brand names of "Bactekiller" (manufactured by Kanebo Ltd., zeolite-based agent), "Ionpure" (manufactured byIshizuka GlassCo.,Ltd., glass-basedagent), "Novaron" (manufactured by Toagosei Co., Ltd., silver-loaded zirconium silver phosphate agent), "Zeomic" (manufactured by Shinagawa Nenryo Co., Ltd., silver-zeolite-based agent), "Zeoplus" (manufactured by Matsumoto Yushi-seiyaku Co., Ltd., silver-zeolite-based agent), "Ceramics AM15" (manufactured by Sumitomo Osaka Cement Co., Ltd., silver-based agent), and "GIN-Tech" (manufactured by Kawasumi Giken Co., Ltd., silver-coated titanium oxide-based agent). The methods for adding silver to these products are roughly divided into a method in which silver ions are adsorbed by an adsorbent such as zeolite, a method in which a silver powder is dispersed in a solvent such as glaze, a method in which a silver compound is reduced electrically or by using an agent (including plating) and the like, and these methods are all on the basis of liquid phase methods.

Examples of a substance to which an antibacterial agent is added include natural fiber, chemical fiber, ceramics, plastics and metals. In the total sales of these various antibacterial processed products, the sale of textile products occupies about a half of the total sales in fiscal 1996 (according to "Report of Discussion Meeting on New Function Processed Products for Daily Use (Antibacterial Processed Products)" issued by the Ministry of Economy, Trade and Industry in December 1998), and there are increasing demands for antibacterial fiber.

As a method for adding an antibacterial agent to fiber, a "kneading method" in which an antibacterial agent is kneaded in fiber at the stage of spinning the fiber or a "post-processing method" in which an antibacterial agent is added to or coated on the surface of fiber by using a processing liquid containing the antibacterial agent at the stage where the fiber is formed into a product in the shape of, for example, cotton, thread or fabric is generally employed.

However, principally a portion of the antibacterial agent present on the surface of fiber actually exhibits the antibacterial function. Therefore, in antibacterial fiber produced by the kneading method, the antibacterial effect is poor considering the added amount, since a portion of the antibacterial agent kneaded inside the fiber does not make a large contribution to the antibacterial effect. When a larger amount of antibacterial agent is kneaded in order to improve the antibacterial effect, it is apprehended that the additional amount can harmfully affect the fiber, for example, the material of the fiber may be changed in its properties, and in addition, there arises a problem of increase of the cost.

In contrast, the post-processing method is advantageous from the viewpoint of the cost and is applicable to a wider range of fibers. Hence it is preferred to the kneading method. In antibacterial fiber processed by this method, most of the antibacterial agent is adhered to the surface of the fiber, and therefore, the fiber exhibits very high antibacterial performance before washing. When it is repeatedly washed, however, the adhered antibacterial agent is peeled off, and therefore, the antibacterial performance attained after repeated washing is largely degraded with the exception of some organic antibacterial agents (that are chemically bonded to a reaction group of fiber). Therefore, in order to retain the antibacterial performance after repeated washing, namely, in order to improve the resistance against washing, the amount of antibacterial agent to be added is increased or a resin binder is used. An inorganic antibacterial agent in particular needs a resin binder. When the amount of antibacterial agent to be added is increased or a resin binder is used, however, the cost is increased, and hence, the advantage in the cost to the kneading method is spoiled as well as there arises a problem that processing characteristics of the post-processing or the texture of a resultant product is degraded.

Accordingly, as methods for overcoming the aforementioned problem of the post-processing method, the following methods have been proposed as a method for producing antibacterial fiber without using a resin binder: A method in which titanium dioxide carrying silver is applied to the surface of fiber by a post-processing method employing an exhaustion dyeing method, a pad dyeing method or a spray dyeing method (disclosed in Patent Document 1); and a method in which silver aerosol is allowed to contact with fiber (disclosed in Patent Document 2).
Patent Document 1: JP 10-280270A
Patent Document 2: JP2005-126348A

### Disclosure of the Invention

### Problems to be Solved by the Invention

With respect to the technique disclosed in Patent Document 1, however, since a processing machine, a dyeing machine and the like are used, large scale equipment is required, given temperature control is necessary and hence, there arises a problem of high cost. There also arises a problem of much time and effort, since an antibacterial agent is produced before applying it to fiber. On the other hand, with respect to the technique disclosed in Patent Document 2, since the production is performed in a nitrogen stream atmosphere or in nitrogen gas stream, there arises a problem that expensive equipment is required and given atmosphere control is difficult, and since silver aerosol is produced before allowing it to contact with fiber, there arises a problem of much time and effort.

Therefore, in consideration of the aforementioned problems of the conventional post-processing method, an object of the invention is to provide an antibacterial treatment method for fiber in which antibacterial metal particulates are firmly adhered to the surface of fiber easily at low cost without using a resin binder for providing antibacterial fiber with high resistance against washing, a method for producing the antibacterial fiber and an antibacterial fiber obtained by the production method.

### Means for Solving the Problems

In consideration of the aforementioned problems, the present inventor has conducted extensive studies and found the following, resulting in achieving the present invention: Antibacterial metal particulates are firmly adhered to fiber by a very simple method in which an aqueous solution containing a noble metal ion or a noble metal complex with fiber immersed therein is irradiated with γ rays or electron rays, and the thus obtained fiber exhibits an antibacterial property with high resistance against washing.
The inventions of respective claims will now be described.

The invention according to claim 1 is an antibacterial treatment method for fiber in which fiber is immersed in an aqueous solution containing a noble metal ion or a noble metal complex, and the aqueous solution is irradiated with γ rays or electron rays so as to cause deposition of noble metal nanoparticles in the vicinity of fiber surface and to make the deposited noble metal nanoparticles to adhere to the surface of the fiber.

In this invention, the noble metal ion or the noble metal complex contained in the aqueous solution is reduced, through the irradiation with the γ rays or the electron rays, into an atomic noble metal on the surface of the fiber, so as to cause deposition of noble metal particulates of nano-order, namely, noble metal nanoparticles. Since the noble metal nanoparticles are firmly bonded to the fiber, the noble metal nanoparticles with an antibacterial property can be firmly adhered to the fiber simply at low cost without using a resin binder. Hence, antibacterial fiber with high resistance against washing can be provided at low cost.

When metal nanoparticles are synthesized and made to adhere to fiber within an aqueous solution, it is generally regarded that the metal nanoparticles cannot be firmly fixed although they are adsorbed onto the surface of the fiber. Accordingly, as a method for making metal nanoparticles adhere firmly to fiber, it is not general to consider a method of synthesizing metal nanoparticles and making them adhere to fiber within an aqueous solution. In the antibacterial treatment method for fiber of this invention, however, the reduction reaction of the noble metal ion and the deposition reaction of the nanoparticles are very uniformly proceeded in the aqueous solution. Hence, it is presumed that the deposition of the noble metal nanoparticles is caused in the extreme vicinity of the fiber immersed in the aqueous solution, and therefore, it seems that a state where the noble metal nanoparticles are firmly fixed onto the fiber may be attained. In other words, it seems that the metal nanoparticles in a very unstable state attained immediately after the deposition come into contact with the surface of the fiber, so as to firmly bond to the surface by utilizing the surface energy. Therefore, although the method for synthesizing the metal nanoparticles and making them adhere to the fiber within the aqueous solution is employed, the metal nanoparticles can be firmly adhered to the fiber.

In the present invention, the noble metal is employed because it is chemically stable and good at antibacterial performance. Such a noble metal is deposited in the form of noble metal nanoparticles and firmly adhered to the fiber having a fine surface so as to attain a large surface area, resulting in increasing possibility of contact with fungi for effectively exhibiting the antibacterial performance.

As described above, the size of the noble metal nanoparticles deposited to be adhered to the fiber is nano-order. The size of the noble metal particles can be controlled by adjusting the concentration of the aqueous solution containing the noble metal ion or the noble metal complex or adjusting the absorbed dose of the γ rays or the electron rays. The size of the noble metal particles is preferably 1 through 500 nm and more preferably 1 through 100 nm. When the size of the noble metal nanoparticles falls within this range, they are more firmly adhered to the fiber and a large surface area is attained, and therefore, the resultant antibacterial fiber achieve higher antibacterial performance .

The aqueous solution containing the noble metal ion used in this invention can be easily obtained by dissolving, in water, inorganic salt such as nitrate, halide (chloride in particular), sulfate or carbonate of a noble metal, or hydrophilic organic acid salt such as acetate or citrate of a noble metal. In particular, nitrate or chloride is preferred because it is easily dissolved in water and is comparatively inexpensive.

The aqueous solution containing the noble metal complex can be easily obtained by coordinating an appropriate ligand to the noble metal ion. The ligand is not particularly specified as far as it has a lone pair or negative charge and may be selected from various kinds of ligands. Specifically, a monodentate ligand such as a halide ion (F⁻, Cl⁻, Br⁻, I⁻or the like), a cyanide ion (CN⁻), ammonia (NH₃) or pyridine, a bidentate ligand such as ethylenediamine (H₂NCH₂CH₂NH₂), an acetyl acetone ion or the like, or a hexadentate ligand such as an ethylenediaminetetraacetic acid ion may be used.

Although an alcohol solution using alcohol such as ethanol or methanol can be used instead of the aqueous solution, the aqueous solution is most preferred in consideration of inconvenience in adjustment of a processing solution, disposal of the processing solution after the antibacterial treatment and the like and the cost for obtaining the alcohol.

A transition-metal ion or a transition-metal complex may be mixed in the aqueous solution containing the noble metal ion or the noble metal complex. When it is mixed, the amount of noble metal ion or noble metal complex can be decreased. In other words, in preparation of the aqueous solution, a part of an expensive noble metal compound can be replaced with an inexpensive transition-metal compound. As a result, the cost can be further lowered. As the transition-metal, copper or nickel is preferably used. Examples of the transition-metal compound include copper sulfate, nickel sulfate, copper nitrate and nickel nitrate.

Conceivable examples of radiation used for depositing the noble metal nanoparticles through irradiation include α rays or β rays emitted from an atomic nucleus, γ rays emitted through change in the energy state of an atomic nucleus, proton rays or electron rays generated by an accelerator, and neutron rays generated by utilizing a nuclear reactor, an accelerator or the like. Such radiation causes an ionization reaction so as to deposit the noble metal nanoparticles from the solution containing the noble metal ion or the noble metal complex. As a result of examination made on the radiation to be employed in this invention, it has been found that the γ rays or the electron rays are suitably used because they do not activate the fiber or the metal particles while causing the ionization reaction. The γ rays and the electron rays are already widely used in sterilization or the like for medical instruments and can be obtained by utilizing existing equipment or techniques.
The wavelength of the radiation used in the invention is preferably less than 1 nm, more preferably 0.1 nm or less and most preferably 0.01 nm or less. As the wavelength is shorter, fine noble metal nanoparticles with a homogenous size tend to be deposited in shorter time.

In order to keep homogeneous dispersion of the noble metal nanoparticles in the fiber, the irradiation of the γ rays or the electron rays is preferably performed with stirring the aqueous solution. The temperature condition is not particularly specified. The irradiation is generally performed at room temperature (ordinary temperature), but cooling conditions or heating conditions of approximately 0 through 100°C may be employed.

The volume of a vessel for the aqueous solution to be irradiated with the γ rays or the electron rays is not particularly specified as far as the aqueous solution accommodated therein can be kept in a homogeneous state by, for example, stirring and the fiber contained therein can be irradiated with the γ rays or the electron rays. The volume of the vessel may be appropriately selected in consideration of the type of aqueous solution, the size of a textile product to be treated, the transmission of the γ rays or the electron rays and the like, and is generally selected from a range of approximately 1 mL through 100 L.

The fiber to which the noble metal particles are adhered in the invention is not particularly specified. Examples of the fiber include natural fiber such as cotton, wool, silk, linen or camel hair, chemical fiber such as rayon or acetate, synthetic fiber such as polyester, polyamide, polyacrylonitrile, polyethylene, polypropylene, polyether imide, polyphenylene sulfide, polyether sulfone, polyether ether ketone or polybenzimidazole, and fabric manufactured by spinning and weaving one of or a mixture of these fibers. Also, the fiber may be short fiber or long fiber, and the fiber may be in the form of cotton or thread, or a product such as a cloth or a knit.

Since the noble metal nanoparticles with a fine particle size adhere to the fiber without using a resin binder in this invention, change in the texture can be suppressed. Furthermore, the degree of coloring the fiber can be adjusted by adjusting the amount of metal nanoparticles adhered to the fiber. At this point, the adjustment is easy because there is no need to use a resin binder. Moreover, since the resultant fiber attains high resistance against washing, change in the texture through washing can be suppressed to be small.

The invention according to claim 2 is the antibacterial treatment method for fiber according to claim 1 in which a noble metal used for making up the noble metal ion or the noble metal complex is at least one noble metal selected from the group consisting of gold, silver, platinum, palladium, ruthenium, rhodium, iridium and rhenium.

In the invention of claim 2, as the noble metal contained in the aqueous solution of the noble metal ion or the noble metal complex, gold, silver, platinum, palladium, ruthenium, rhodium, iridium or rhenium is preferably used from the viewpoint of high antibacterial performance, oxidation resistance and the like. Among these noble metals, silver is most preferred from the viewpoint of the antibacterial performance and the cost.

As described above, the aqueous solution containing the noble metal ion or the noble metal complex can be obtained by dissolving an inorganic salt or a hydrophilic organic acid salt of a noble metal in water. With respect to the aforementioned noble metals, specifically, silver nitrate, chloroauric acid, palladium chloride, silver sulfate or the like is used.
Among what is called noble metal elements, osmium is excluded from the invention because its oxide is very venomous.

The invention according to claim 3 is the antibacterial treatment method for fiber according to claim 1 or 2 in which the concentration of the noble metal ion or the noble metal complex in the aqueous solution containing the noble metal ion or the noble metal complex is 1 µM through 1M.

In the invention of claim 3, from the viewpoint of the antibacterial performance, the size of the noble metal nanoparticles to be deposited and the like, the concentration of the aqueous solution containing the noble metal ion or the noble metal complex is preferably 1 µM through 1 M. More preferably, the concentration is 0.1 through 10 mM.

The invention according to claim 4 is the antibacterial treatment method for fiber according to claim 1 or 2 in which the noble metal is silver and the concentration of a silver ion or a silver complex in the aqueous solution is 0.005 through 10 mM.

When a silver ion aqueous solution or an aqueous solution containing a silver complex is prepared to have a concentration of 0.005 through 10 mM and the fiber immersed in this aqueous solution is irradiated with the γ rays or the electron rays, a textile product with a higher antibacterial property and higher resistance against washing can be obtained.

The invention according to claim 5 is the antibacterial treatment method for fiber according to claim 4 in which the noble metal is silver and the concentration of a silver ion or a silver complex in the aqueous solution is 0.01 through 10 mM.

When a silver ion aqueous solution or an aqueous solution containing a silver complex is prepared to have a concentration of 0.01 through 10 mM and the fiber immersed in this aqueous solution is irradiated with the γ rays or the electron rays, a textile product with a much higher antibacterial property and much higher resistance against washing can be obtained.

The invention according to claim 6 is the antibacterial treatment method for fiber according to any one of claims 1 through 5 in which the γ rays or the electron rays are irradiated under a condition of an absorbed dose of 1 J/kg or more.

In order to definitely cause the reduction reaction of the noble metal ion, the γ rays or the electron rays are irradiated preferably under the condition of an absorbed dose of 1 J/kg (= 1 Gy) or more. Also, in order to suppress the harmful effect of the γ rays or the electron rays on the fiber for preventing the change in the texture, the absorbed dose is preferably 1,000,000 J/kg or less. The absorbed dose is more preferably 1 through 1,000,000 J/kg, and most preferably 100 through 100,000 J/kg.

In particular, when the γ rays are used as the radiation, the γ rays are irradiated preferably under a condition of a dose of 1 Gy or more. As a specific preferable example of the γ irradiation, a cobalt-60 γ radiation source (with energy of γ ray photon of 1.25 MeV) is used as a radiation source, so as to perform the irradiation under conditions of a dose rate of approximately 3 kGy/h and irradiation time of 1 through 18 hours.

The invention according to claim 7 is the antibacterial treatment method for fiber according to any one of claims 1 through 5 in which the noble metal is silver and the irradiation with the γ rays or the electron rays is performed under a condition of an absorbed dose of 10 through 50000 J/kg.

When the fiber immersed in a silver ion aqueous solution or an aqueous solution containing a silver complex is irradiated with the γ rays or the electron rays at an absorbed dose of 10 through 50000 J/kg, a textile product with a higher antibacterial property and higher resistance against washing can be obtained.

The invention according to claim 8 is the antibacterial treatment method for fiber according to any one of claims 1 through 7 in which the aqueous solution containing the noble metal ion or the noble metal complex further comprises at least one additive selected from the group consisting of a water-soluble polymer, a surface active agent and an organic solvent.

In the invention of claim 8, when at least one additive selected from the group consisting of a water-soluble polymer, a surface active agent and an organic solvent is added to the aqueous solution containing the noble metal ion or the noble metal complex, the rate of the reduction reaction of the noble metal ion can be increased, or the size or the like of the noble metal nanoparticles to be deposited can be adjusted.

When irradiated with the radiation, through collision with a photon, or the like, with very high energy the additive produces a radical. It seems that this radical increases the rate of the reduction reaction of the noble metal ion or adjusts the size or the like of the particles.

Preferable examples of the additive include a water-soluble polymer such as polyvinyl alcohol, a surface active agent, alcohols, ethers such as tetrahydrofuran, diethyl ether and diisopropyl ether, polyols such as alkylene glycol, polyalkylene glycol, monoalkyl ether or dialkyl ether of these glycols, and glycerin, carboxylic acids such as formic acid, acetic acid, propionic acid, lactic acid and glycolic acid, and ketones such as acetone and methyl ethyl ketone.

The invention according to claim 9 is a method for producing antibacterial fiber comprising the antibacterial treatment method for fiber according to any one of claims 1 through 8.

According to the invention of claim 9, noble metal nanoparticles with an antibacterial property can be firmly adhered to the fiber easily at low cost without using a resin binder. Hence, antibacterial fiber with high resistance against washing can be produced at low cost.

### Effects of the Invention

According to the present invention, antibacterial metal nanoparticles can be made to firmly adhere to the surface of fiber easily at low cost without using a resin binder, and hence, antibacterial fiber with high resistance against washing can be provided.

### Best Modes for Carrying Out the Invention

Now, an embodiment of the invention will be described with reference to examples. It is noted that the invention is not limited to the embodiment described below. It is possible to make various modifications on the embodiment described below within a range the same as or equivalent to the present invention.

### 1. Example 1 and Comparative Example 1

In Example 1 and Comparative Example 1, silver was used as the noble metal for the antibacterial treatment, and the thus attained antibacterial performance was compared with that of a commercially available cloth of antibacterial silver fiber.

### a. Example 1

### (1) Preparation of Antibacterial Fiber Sample

A silver nitrate aqueous solution obtained by adding silver nitrate to 800 mL of water so as to attain a concentration of 0.1 mM was put in a 1-L plastic bottle, and 8 mL of 2-propanol was further added thereto for accelerating the reduction reaction of silver ions. In the thus obtained aqueous solution, 8.6 mg of silver is present in the form of ions.

A commercially available cotton cloth (with a size of 50 cm x 50 cm) was immersed in the resultant aqueous solution, and the plastic bottle was sealed and irradiated with cobalt-60 γ rays by using cobalt-60 γ irradiation equipment with stirring under the following conditions:
Radiation Source: Cobalt-60 γ source (with energy of γ ray photon of 1.25 MeV)
Source Strength: approximately 7000 curie
Dose Rate: 3 kGy/h
Irradiation Time: 2 hours

Thereafter, the cotton cloth was taken out of the plastic bottle, washed with water and dried, and thus, an antibacterial fiber sample of Example 1 having silver nanoparticles adhered to the surface of fiber was obtained.

### b. Comparative Example 1

A commercially available cloth of antibacterial silver fiber (trade name: Ag fresh processing, manufactured by Nisshin Boseki Co., Ltd.) was used as an antibacterial fiber sample of Comparative Example 1 without subjecting it to any particular treatment such as irradiation with the γ rays.

### c. Performance Evaluation (Antibacterial Performance Test)

Each sample obtained in Example 1 and Comparative Example 1 was divided into two pieces, one of which was directly used as a sample (hereinafter referred to as a "sample with 0 washing") and the other of which was washed 10 times (hereinafter referred to as a "sample with 10 washing"). The washing was performed in accordance with the attached table No. 103 for line dry of JIS L0217 (Care Symbols and Care Labeling for Handling of Textiles), and a JAFET standard detergent (with a neutral property; a nonionic surface active agent) was used as a detergent.

Thereafter, each of the samples (i.e., the sample with 0 washing and the sample with 10 washing) was subjected to an antibacterial test. The antibacterial test was performed by inoculating a prescribed number of fungi (with a viable cell count [A]) in each sample and measuring a viable cell count [C] attained after cultivation of 18 hours, and the antibacterial performance is obtained by comparing the viable cell count [C] with a viable cell count [B] attained after the cultivation of 18 hours in a non-processed sample (i.e., a standard cloth) not subjected to an antibacterial treatment. (Specifically, a difference in a logarithm value between [B] and [C] is expressed as a bacteriostatic activity value: Bacteriostatic activity value = log [B] - log [C].) This test method is specifically provided in JIS L1902 (Antibacterial Test Method for Textiles (quantitative method: bacterial culture absorbing method), and Staphyloccus aureus ATCC 6538P (Yellow staph) was used as the test fungi.

In the aforementioned test, the test fungi were inoculated by dropping, onto each sample, a test fungi suspension containing 0.05% of a nonionic surface active agent, and the viable cell count was measured by pour plate method. The test was carried out in Japan Textile Products Quality and Technology Center (which also applies to examples described below).
The test results are shown in Table 1 together with data of the non-processed sample.

**[Table 1]**

| | | | | |
|---|---|---|---|---|
| Non-processed sample | immediately after inoculation, Log [A] | | 4.2 | |
| | after cultivation of 18 hours, Log [B] | | 7.5 | |
| | | | | |

| Sample | | Bacteriostatic activity value | | |
|---|---|---|---|---|
| Example 1 | | 0 washing | | 4.7 or more |
| | | After 10 washing | | 4.6 |
| Comparative Example 1 | | 0 washing | | 4.7 or more |
| | | After 10 washing | | 3.2 |

It is understood from Table 1 that the resistance against washing is better in Example 1 than in Comparative Example 1.

### 2. Examples 2 through 5

In the following examples, silver ions were used as the noble metal ions, and the relationship between the concentration and the antibacterial property was tested.

### (1) Preparation of Samples

A silver nitrate aqueous solution obtained by adding silver nitrate to 1 L of water so as to attain each concentration shown in Table 2 was put in a vessel, and 1 vol% of 2-propanol was further added thereto for accelerating the reduction reaction of silver ions.

A commercially available cotton cloth (with a size of 50 cm x 50 cm) was immersed in each of the resultant aqueous solutions, and the vessel was sealed and irradiated with cobalt-60 γ rays at an absorbed dose of 10 kGy (10000 J/kg) by using the same cobalt-60 γ irradiation equipment as in Example 1. It is noted that the aqueous solution was not stirred during the irradiation. Thereafter, the cotton cloth was taken out of the vessel, washed with water and dried to obtain a sample of each of Examples 2 through 5 having silver nanoparticles adhered to the surface of the fiber.

### (2) Performance Evaluation (Antibacterial Performance Test)

Each of the samples of Examples 2 through 5 thus obtained was subjected to the antibacterial performance test in the same manner as in Example 1. The test results are shown in Table 2 together with the data of the non-processed sample.

**[Table 2]**

| | | | | |
|---|---|---|---|---|
| Non-processed sample | | immediately after inoculation, Log [A] | | 4.1 |
| | | after cultivation of 18 hours, Log [B] | | 7.2 |
| | | | | |

| Example | Concentration of silver nitrate (silver ions) (mM) | | Bacteriostatic activity value | |
|---|---|---|---|---|
| | | | 0 washing | After 10 washing |
| 2 | 0.01 | | 4.4 or more | 2.7 |
| 3 | 0.1 | | 4.4 or more | 4.1 |
| 4 | 1 | | 4.4 or more | 4.3 |
| 5 | 10 | | 4.4 or more | 4.2 |

It is understood from the test results shown in Table 2 that the samples of all Examples 2 through 5 are good at the antibacterial performance because their bacteriostatic activity values obtained with 0 washing were all more than 4. Also, when the silver ion concentration is 0.1 mM or more (as in Examples 3 through 5), the bacteriostatic activity values were more than 4 even after 10 washing, and thus, it is understood that the samples of these examples are good at the resistance against washing. When the silver ion concentration is 0.01 mM (as in Example 2), the bacteriostatic activity value attained after 10 washing is lowered to 2.7, but it is still a satisfactory value as the antibacterial performance.

### 3. Examples 6 through 10

In the following examples, silver ions were used as the noble metal ions, and the relationship between the absorbed dose and the antibacterial property was tested.

### (1) Preparation of Samples

A silver nitrate aqueous solution obtained by adding silver nitrate to 1 L of water was put in a vessel, and 1 vol% of 2-propanol was further added thereto for accelerating the reduction reaction of silver ions.

A commercially available cotton cloth (with a size of 50 cm x 50 cm) was immersed in the resultant aqueous solution, and the vessel was sealed and irradiated with cobalt-60 γ rays at each absorbed dose shown in Table 3 by using the same cobalt-60 γ irradiation equipment as in Example 1. It is noted that the aqueous solution was not stirred during the irradiation. Thereafter, the cotton cloth was taken out of the vessel, washed with water and dried to obtain a sample of each of Examples 6 through 10 having silver nanoparticles adhered to the surface of the fiber.

### (2) Performance Evaluation (Antibacterial Performance Test)

Each of the samples of Examples 6 through 10 thus obtained was subjected to the antibacterial performance test in the same manner as in Example 1. The test results are shown in Table 3 together with the data of the non-processed sample.

**[Table 3]**

| | | | | | |
|---|---|---|---|---|---|
| Non-processed sample | | immediately after inoculation, Log [A] | | | 4.1 |
| | | after cultivation of 18 hours, Log [B] | | | 7.1 |
| | | | | | |

| Example | Absorbed dose (J/kg) | | Bacteriostatic activity value | | |
|---|---|---|---|---|---|
| | | | 0 washing | After 10 washing | |
| 6 | 10 | | 4.3 or more | 4.2 | |
| 7 | 100 | | 4.3 or more | 4.3 or more | |
| 8 | 1000 | | 4.3 or more | 4.3 or more | |
| 9 | 10000 | | 4.3 or more | 4.3 or more | |
| 10 | 100000 | | 4.3 or more | 4.3 or more | |

It is understood from the test results shown in Table 3 that the samples of Examples 6 through 10 are good at the antibacterial performance and the resistance against washing even when the absorbed dose is small because their bacteriostatic activity values are all more than 4 with 0 washing and after 10 washing. When the absorbed dose was 100000 J/kg (100 kGy) (as in Example 10), the texture (specifically, the appearance and the feel) of the fiber was slightly changed.

### 4. Example 11

In this example, gold was used as the noble metal instead of silver, and the influence of the difference in the kind of noble metal on the antibacterial performance was tested.

### (1) Preparation of Samples

A gold ion aqueous solution with a gold ion concentration of 0.1 mM is prepared from 1 L of water, and the solution was put in a vessel. Thereto, 1 vol% of 2-propanol was further added for accelerating the reduction reaction of gold ions.

A commercially available cotton cloth (with a size of 50 cm x 50 cm) was immersed in the resultant aqueous solution, and the vessel was sealed and irradiated with cobalt-60 γ rays at an absorbed dose of 1 kGy (1000 J/kg) by using the same cobalt-60 γ irradiation equipment as in Example 1. It is noted that the aqueous solution was not stirred during the irradiation. Thereafter, the cotton cloth was taken out of the vessel, washed with water and dried to obtain a sample of Example 11 having gold nanoparticles adhered to the surface of the fiber.

### (2) Performance Evaluation (Antibacterial Performance Test)

The sample of Example 11 thus obtained was subjected to the antibacterial performance test in the same manner as in Example 1. As a result, a bacteriostatic activity value of 1.6 was attained with 0 washing and a bacteriostatic activity value of 0.9 was attained after 10 washing. Thus, it was found that the antibacterial performance was not as high as that attained by using silver (as in Example 8).

As described so far, by the simple and low-cost antibacterial treatment method for fiber of this invention in which fiber is immersed in an aqueous solution containing a noble metal ion or a noble metal complex and the aqueous solution is irradiated with γ rays or electron rays, antibacterial fiber with high resistance against washing can be obtained at low cost.

## Claims

1. An antibacterial treatment method for fiber in which fiber is immersed in an aqueous solution containing a noble metal ion or a noble metal complex, and the aqueous solution is irradiated with γ rays or electron rays so as to cause deposition of noble metal nanoparticles in the vicinity of fiber surface and to make the deposited noble metal nanoparticles to adhere to the surface of the fiber.

2. The antibacterial treatment method for fiber according to claim 1 in which a noble metal used for making up the noble metal ion or the noble metal complex is at least one noble metal selected from the group consisting of gold, silver, platinum, palladium, ruthenium, rhodium, iridium and rhenium.

3. The antibacterial treatment method for fiber according to claim 1 or 2 in which the concentration of the noble metal ion or the noble metal complex in the aqueous solution containing the noble metal ion or the noble metal complex is 1 µM through 1M.

4. The antibacterial treatment method for fiber according to claim 1 or 2 in which the noble metal is silver and the concentration of a silver ion or a silver complex in the aqueous solution is 0.005 through 10 mM.

5. The antibacterial treatment method for fiber according to claim 4 in which the noble metal is silver and the concentration of a silver ion or a silver complex in the aqueous solution is 0.01 through 10 mM.

6. The antibacterial treatment method for fiber according to any one of claims 1 through 5 in which the γ rays or the electron rays are irradiated under a condition of an absorbed dose of 1 J/kg or more.

7. The antibacterial treatment method for fiber according to any one of claims 1 through 5 in which the noble metal is silver and the irradiation with the γ rays or the electron rays is performed under a condition of an absorbed dose of 10 through 50000 J/kg.

8. The antibacterial treatment method for fiber according to any one of claims 1 through 7 in which the aqueous solution containing the noble metal ion or the noble metal complex further comprises at least one additive selected from the group consisting of a water-soluble polymer, a surface active agent and an organic solvent.

9. A method for producing antibacterial fiber comprising the antibacterial treatment method for fiber according to any one of claims 1 through 8.

## Patentansprüche

1. Ein antibakterielles Behandlungsverfahren für eine Faser, in welcher die Faser in eine wässrige Lösung, enthaltend ein Edelmetallion oder einen Edelmetallkomplex, eingebracht wird und die wässrige Lösung mit Röntgenstrahlen oder Elektronenstrahlen bestrahlt wird, um so Abscheidung von Edelmetallnanopartikeln in der Umgebung einer Faseroberfläche hervorzurufen und um die abgeschiedenen Edelmetallnanopartikel an der Oberfläche der Faser haften zu lassen.

2. Antibakterielles Behandlungsverfahren für eine Faser einer Faser nach Anspruch 1, wobei ein Edelmetall zur Herstellung des Edelmetallions oder des Edelmetallkomplexes wenigstens ein Edelmetall ist, ausgewählt aus der Gruppe bestehend aus Gold, Silber, Platin, Palladium, Ruthenium, Rhodium, Iridium und Rhenium.

3. Antibakterielles Behandlungsverfahren für eine Faser nach Anspruch 1 oder 2, bei welchem die Konzentration des Edelmetallions oder des Edelmetallkomplexes in der ein Edelmetallion oder einen Edelmetallkomplex enthaltenden wässrigen Lösung 1 µM bis 1 M ist.

4. Antibakterielles Behandlungsverfahren für eine Faser nach Anspruch 1 oder 2, bei welchem das Edelmetall Silber ist und die Konzentration eines Silberions oder eines Silberkomplexes in der wässrigen Lösung 0,005 bis 10 mM ist.

5. Antibakterielles Behandlungsverfahren für eine Faser nach Anspruch 4, bei welchem das Edelmetall Silber ist und die Konzentration eines Silberions oder eines Silberkomplexes in der wässrigen Lösung 0,01 bis 10 mM ist.

6. Antibakterielles Behandlungsverfahren für eine Faser nach einem der Ansprüche 1 bis 5, bei welchem die Röntgenstrahlen oder die Elektronenstrahlen eingestrahlt werden unter einer Bedingung einer absorbierten Dosis von 1J/kg oder mehr.

7. Antibakterielles Behandlungsverfahren für eine Faser nach einem der Ansprüche 1 bis 5, bei welchem das Edelmetall Silber ist und die Bestrahlung mit den Röntgenstrahlen oder den Elektronenstrahlen unter einer Bedingung einer absorbierten Dosis von 10 bis 50000 J/kg durchgeführt wird.

8. Antibakterielles Behandlungsverfahren für eine Faser nach einem der Ansprüche 1 bis 7, bei welchem die ein Edelmetallion oder einen Edelmetallkomplex enthaltende wässrige Lösung weiterhin wenigstens ein Additiv umfasst, welches ausgewählt ist aus der Gruppe bestehend aus einem wasserlöslichen Polymer, einem oberflächenaktiven Mittel und einem organischen Lösungsmittel.

9. Antibakterielles Behandlungsverfahren für eine Faser nach dem Verfahren zur antibakteriellen Behandlung einer Faser nach einem der Ansprüche 1 bis 8.

## Revendications

1. Procédé de traitement antibactérien pour fibre, dans lequel une fibre est immergée dans une solution aqueuse contenant un ion de métal noble ou un complexe de métal noble, et la solution aqueuse est irradiée par des rayons γ ou des faisceaux d'électrons afin de donner lieu à un dépôt de nanoparticules de métal noble au voisinage de la surface de fibre et de faire adhérer les nanoparticules de métal noble déposées à la surface de la fibre.

2. Procédé de traitement antibactérien pour fibre selon la revendication 1, dans lequel un métal noble utilisé pour constituer l'ion de métal noble ou le complexe de métal noble est au moins un métal noble choisi parmi le groupe consistant en l'or, l'argent, le platine, le palladium, le ruthénium, le rhodium, l'iridium et le rhénium.

3. Procédé de traitement antibactérien pour fibre selon la revendication 1 ou 2, dans lequel la concentration de l'ion de métal noble ou du complexe de métal noble dans la solution aqueuse contenant l'ion de métal noble ou le complexe de métal noble est de 1 µM à 1 M.

4. Procédé de traitement antibactérien pour fibre selon la revendication 1 ou 2, dans lequel le métal noble est l'argent et la concentration d'un ion argent ou d'un complexe d'argent dans la solution aqueuse est de 0,005 à 10 mM.

5. Procédé de traitement antibactérien pour fibre selon la revendication 4, dans lequel le métal noble est l'argent et la concentration d'un ion argent ou d'un complexe d'argent dans la solution aqueuse est de 0,01 à 10 mM.

6. Procédé de traitement antibactérien pour fibre selon l'une quelconque des revendications 1 à 5, dans lequel les rayons γ ou les faisceaux d'électrons sont irradiés dans une condition d'une dose absorbée de 1 J/kg ou plus.

7. Procédé de traitement antibactérien pour fibre selon l'une quelconque des revendications 1 à 5, dans lequel le métal noble est l'argent et l'irradiation avec les rayons γ ou les faisceaux d'électrons est réalisée dans une condition d'une dose absorbée de 10 à 50 000 J/kg.

8. Procédé de traitement antibactérien pour fibre selon l'une quelconque des revendications 1 à 7, dans lequel la solution aqueuse contenant l'ion de métal noble ou le complexe de métal noble comprend en outre au moins un additif choisi parmi le groupe consistant en un polymère soluble dans l'eau, un agent tensioactif et un solvant organique.

9. Procédé de production d'une fibre antibactérienne comprenant le procédé de traitement antibactérien pour fibre selon l'une quelconque des revendications 1 à 8.
